# EUROPEAN PATENT SPECIFICATION

(11) **EP 1 697 765 B1**
(45) Date of publication and mention of the grant of the patent: **21.11.2012**
(21) Application number: 04801491.4
(22) Date of filing: 07.12.2004
(51) Int. Cl.: G01S 15/89, G01S 7/52, A61B 8/14

(54) **ULTRASONIC DIAGNOSTIC CONTRAST IMAGING WITH SPATIAL COMPOUNDING**
DIAGNOSTISCHE ULTRASCHALL-KONTRASTABBILDUNG MIT RÄUMLICHER MISCHUNG
IMAGERIE DE CONTRASTE DIAGNOSTIQUE A ULTRASONS, A COMBINAISON SPATIALE

(30) Priority: 16.12.2003 US 529781 P
(43) Date of publication of application: 06.09.2006
(73) Proprietor: Koninklijke Philips Electronics N.V., 5621 BA Eindhoven (NL)
(72) Inventor: AVERKIOU, Michalakis, Bothell, Washington 98041-3003 (US); JENSEN, Seth, Bothell, Washington 98041-3003 (US)
(74) Representative: Roche, Denis
(86) International application number: PCT/IB2004/052698
(87) International publication number: WO 2005/059591

(56) References cited:
- US-A1- 2002 042 576
- US-A1- 2003 004 414
- US-B1- 6 530 885
- JESPERSEN S K ET AL INSTITUTE OF ELECTRICAL AND ELECTRONICS ENGINEERS: "ULTRASOUND SPATIAL COMPOUND SCANNER FOR IMPROVED VISUALIZATION IN VASCULAR IMAGING" 1998 IEEE ULTRASONICS SYMPOSIUM PROCEEDINGS. SENDAI, MIYAGI, JP, OCT. 5 - 8, 1998, IEEE ULTRASONICS SYMPOSIUM PROCEEDINGS, NEW YORK, NY : IEEE, US, vol. VOL.2, 5 October 1998 (1998-10-05), pages 1623-1626, XP000871850 ISBN: 0-7803-4096-5
- ENTREKIN R ET AL: "Real time spatial compound imaging in breast ultrasound: technology and early clinical experience" MEDICAMUNDI, PHILIPS MEDICAL SYSTEMS, SHELTON, CT,, US, vol. 43, no. 3, September 1999 (1999-09), pages 35-43, XP002181821 ISSN: 0025-7664

## Description

This invention relates to medical diagnostic imaging systems and, in particular, to ultrasonic diagnostic imaging systems which image contrast agents within the body by spatial compounding.

The ultrasonic imaging of blood flow and tissue vasculature within the body can be greatly enhanced by infusing the bloodstream with an ultrasonic contrast agent. Contrast agents are solutions of suspended microbubbles which are highly reflective of ultrasound. As the microbubbles travel through the vasculature and vessels being imaged, the strong echo reflections from the microbubbles cause the blood stream to "light up" in the ultrasound image, enabling the clinician to more clearly diagnose conditions in the heart and blood vessels. See for example US Pat. 5,833,613 (Averkiou et al.) The high harmonic content of echo signals from microbubbles enables the vasculature to be more easily segmented and displayed against the tissue background. See US Pat. 5,951,478 (Hwang et al.)

US 6 530 885 B1 discloses a spatially compounded three-dimensional ultrasonic image acquisition according to the preambles of claims 1 and 12. US 2002/0042576 A1 discloses ultrasonic non-linear imaging at fundamental frequencies.

Contrast agents can be used in several different ways. One is to insonify the image field with high energy (high MI) ultrasound which disrupts or breaks the microbubbles. The disruption events produce strong nonlinear reflections which can be readily detested and displayed. See US Pat. 5,456,257 (Johnson et al.) But with the new generation of contrast agents currently being developed, the nonlinear response can be elicited without substantial disruption of the microbubbles, enabling more extended imaging sessions on a single contrast agent infusion. In this technique microbubbles can be continuously imaged at low transmit energies (low MI). See US Pat. 6,171,246 (Averkiou et al.)

However, low MI contrast images often suffer from very low signal to noise ratios, compromised axial resolution, and overall image aesthetics. This is due to the desire to use the lowest MI possible for minimal microbubble disruption which reduces the intensity of the returning echoes. These adverse effects are also seen when a multi-pulse scheme is used to separate the nonlinear components of the contrast echoes such as those described in US Pats. 5,706,819 (Hwang) and 5,577,505 (Brock-Fisher et al.) These techniques, referred to as pulse inversion, require the transmission of multiple pulses for harmonic separation. Accordingly it would be desirable to improve the quality of contrast images while retaining the advantage of good image aesthetics.

In accordance with the principles of the present invention, an ultrasonic diagnostic imaging system is provided which performs ultrasonic contrast imaging by spatial compounding. The region of interest is interrogated by beams from a plurality of different look directions and the backscattered signals processed for harmonic separation and combined to produce a spatially compounded harmonic contrast image. The spatial compounding not only reduces image speckle and improves the clarity of arcuate anatomical features, but also improves the signal to noise of low MI images. In several illustrated embodiments component images for spatial compounding are acquired in ways which enable the nonlinear signal content to be separated by pulse inversion. In another embodiment the acquisition rate of spatially compounded harmonic contrast images is improved through pulse inversion separation by interpolation. In yet another embodiment fundamental and harmonic acquisition is interleaved to produce a tissue image with a spatially compounded overlay of contrast enhanced blood flow.

In the drawings:
FIGURE 1 illustrates in block diagram form an ultrasonic diagnostic imaging system which produces spatially compounded contrast images in accordance with the principles of the present invention.
FIGURE 2 illustrates a tissue image with a contrast enhanced overlay of pathology and vasculature.
FIGURES 3a-3c illustrate a technique for acquiring a tissue image with a spatially compounded overlay of contrast enhanced blood flow.
FIGURES 4a-4c illustrate beam profiles for the multiline acquisition of spatially compounded nonlinear images.
FIGURES 5a-5c illustrate component images for spatial compounding which may be acquired by the technique of FIGURES 4a-4c.
FIGURES 6a-6c illustrate a high speed acquisition technique for spatially compounded harmonic contrast imaging.
FIGURE 7 illustrates the combining of component images for spatial compounding and harmonic separation by pulse inversion.

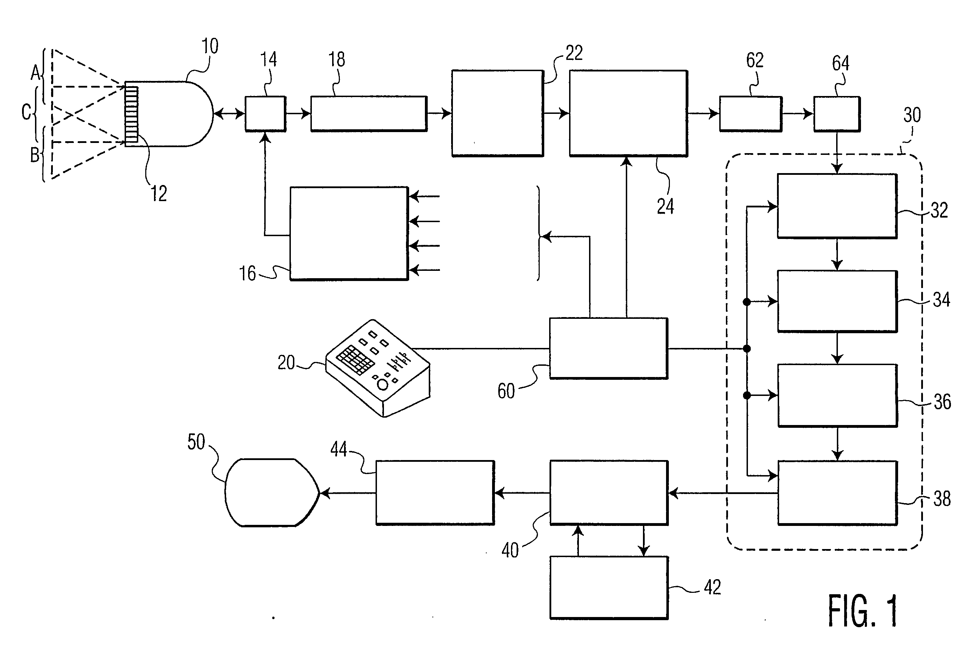

Referring first to FIGURE 1, an ultrasonic diagnostic imaging system constructed in accordance with the principles of the present invention is shown in block diagram form. This system operates by scanning a two or three dimensional region of the body being imaged with ultrasonic transmit beams. A scanhead 10 including an array transducer 12 transmits beams at different angles (look directions) over an image field denoted by the dashed rectangle and parallelograms. Three groups of scanlines are indicated in the drawing, labeled A, B, and C with each group being steered at a different angle relative to the scanhead. The transmission of the beams is controlled by a transmitter 16 which controls the phasing and time of actuation of each of the elements of the array transducer so as to transmit each beam from a predetermined origin along the array and at a predetermined angle. As each beam is transmitted along its steered path through the body, the beam returns echo signals with linear and nonlinear (fundamental and harmonic) components corresponding to the transmitted frequency components. The transmit signals are modulated by the nonlinear response of contrast agent microbubbles encountered by the beam, thereby generating echo signals with harmonic components.

The ultrasound system of FIGURE 1 utilizes a transmitter 16 which transmits waves or pulses of a selected modulation characteristic in a desired beam direction for the return of harmonic echo components from scatterers within the body. The transmitter is responsive to a number of control parameters which determine the characteristics of the transmit beams as shown in the drawing, including the frequency components of the transmit beam, their relative intensities or amplitudes, and the phase or polarity of the transmit signals. The transmitter is coupled by a transmit/receive switch 14 to the elements of the array transducer 12 of the scanhead 10. The array transducer can be a one dimensional array for planar (two dimensional) imaging or a two dimensional array for two dimensional or volumetric (three dimensional) imaging.

The transducer array 12 receives echoes from the body containing fundamental and harmonic frequency components which are within the transducer passband. These echo signals are coupled by the switch 14 to a beamformer 18 which appropriately delays echo signals from the different transducer elements then combines them to form a sequence of fundamental and harmonic signals along the receive beam direction from shallow to deeper depths. Preferably the beamformer is a digital beamformer operating on digitized echo signals to produce a sequence of discrete coherent digital echo signals from a near field to a far field depth of field. The beamformer may be a multiline beamformer which produces two or more sequences of echo signals along multiple spatially distinct receive scanlines in response to a single transmit beam, which is particularly useful for 3D imaging and for high speed acquisition of spatially compounded contrast image as described in several of the following embodiments. The beamformed echo signals are coupled to an ensemble memory 22. The transmitter 16 and the beamformer 18 are operated under control of a system controller 60, which in turn is responsive to the settings of controls on a user interface 20 operated by the user of the ultrasound system. The system controller controls the transmitter to transmit the desired number of scanline groups at the desired angles, transmit energies, frequencies and modulation characteristics. The system controller also controls the digital beamformer to properly delay and combine the received echo signals for the apertures and image depths used.

In accordance with one aspect of the present invention, multiple waves or pulses are transmitted in each beam direction using different modulation techniques, resulting in the reception of multiple echoes for each scanned point in the image field. The echoes corresponding to a common spatial location are referred to herein as an ensemble of echoes, and are stored in the ensemble memory 22, from which they can be retrieved and processed together. The echoes of an ensemble are combined in various ways as described more fully below by the nonlinear signal separator 24 to produce the desired nonlinear or harmonic signals of the contrast agent. This harmonic separation acts to enhance the nonlinear components by reinforcement and suppress the fundamental components by cancellation due to the different transmit modulation of the echo signals being combined. The modulation techniques used can be amplitude modulation, phase modulation, polarity modulation or a combination thereof. See concurrently filed US patent application US 2005/0124895 A1 entitled "ULTRASONIC SPECKLE REDUCTION USING NONLINEAR ECHO COMBINATIONS" for a description of the various ways in which the echoes of differently modulated signals can be combined for nonlinear separation. The separated signals are filtered by a filter 62 to remove unwanted frequency components. When imaging harmonic contrast agents the passband of the filter 62 is set to pass harmonics of the transmit band. The filtered signals are subjected to B mode or Doppler detection by a detector 64. For B mode imaging the detector 24 will perform amplitude detection of the echo signal envelope. For Doppler imaging ensembles of echoes are assembled for each point in the image and are Doppler processed to estimate the Doppler shift or Doppler power intensity.

In accordance with another aspect of the present invention the digital echo signals are processed by spatial compounding in a processor 30. The digital echo signals are initially pre-processed by a preprocessor 32. The pre-processor 32 can preweight the signal samples if desired with a weighting factor. The samples can be preweighted with a weighting factor that is a function of the number of component frames used to form a particular compound image. The pre-processor can also weight edge lines that are at the edge of one overlapping component image so as to smooth the transitions where the number of samples or images which are compounded changes. The pre-processed signal samples may then undergo a resampling in a resampler 34. The resampler 34 can spatially realign the estimates of one component image to those of another component image or to the pixels of the display space.

After resampling the component images are compounded on a spatial basis by a combiner 36. Combining may comprise summation, averaging, peak detection, or other combinational means. The samples being combined may also be weighted prior to combining in this step of the process. Finally, post-processing is performed by a post-processor 38. The post-processor normalizes the combined values to a display range of values. Post-processing can be most easily implemented by look-up tables and can simultaneously perform compression and mapping of the range of compounded values to a range of values suitable for display of the compounded image.

The compounding process may be performed in estimate data space or in display pixel space. In a constructed embodiment scan conversion is done following the compounding process by a scan converter 40. The compound images may be stored in a Cineloop® memory 42 in either estimate or display pixel form. If stored in estimate form the images may be scan converted when replayed from the Cineloop memory for display. The scan converter and Cineloop memory may also be used to render three dimensional presentations of the spatially compounded images as described in U.S. Patents 5,485,842 and 5,860,924. Following scan conversion the spatially compounded images are processed for display by a video processor 44 and displayed on an image display 50.

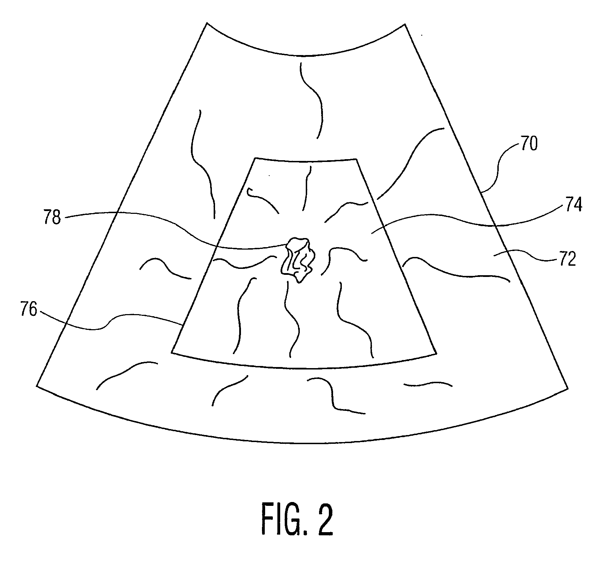

FIGURE 2 illustrates an overlay contrast image 70 of the type described in aforementioned US Pat. 6,171,246 (Averkiou et al.) This image 70 comprises an echo tissue background 72 with an area bounded by 76 which is overlaid with a harmonic contrast image 74 of contrast agent microbubbles. The multiple look directions used to acquire the image signals improve the ability to discern tumors and masses in the image field such as that illustrated at 78. The combination of signals from different look directions improves the signal to noise ratio of the signals returned from the mass 78 enabling it to stand out more clearly against the background, and the different look directions provide better definition of the contours of the mass 78. In addition, subtle masses which may be obscured by image speckle can be easier to discern. The harmonic contrast image can be colored to better highlight the vasculature in the mass.

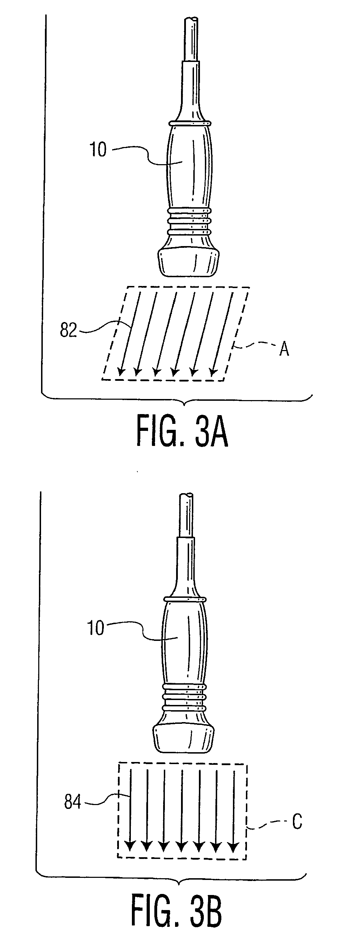

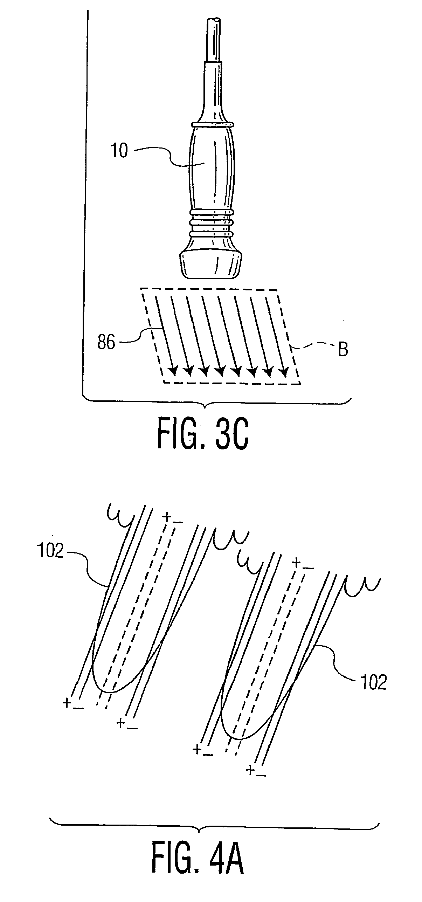

A technique for acquiring this image 70 is illustrated in FIGURES 3a-3c. An ultrasound probe 10 transmits beams 82, 84, 86 into the body at different look directions A, B, and C. This can be done by transmitting all of the beams of a specific look direction before transmitting beams of another look direction. Alternatively it can done by interleaving transmissions of beams of different look directions. It can also be done by transmitting beams in different look directions simultaneously as described in US patent application US 2006/0293596 A1 entitled "ULTRASONIC SPATIAL COMPOUNDING WITH MULTIPLE SIMULTANEOUS BEAM TRANSMISSION." FIGURE 3a shows a group of beams 82 which are transmitted in a look direction A, FIGURE 3b illustrates a group of beams 84 which are transmitted in a look direction C, and FIGURE 3c illustrates a group of beams 86 which are transmitted in a look direction B.

In the embodiment of FIGURES 2 and 3, both fundamental and harmonic signals are used to produce the overlay contrast image 70. One way to do this is to transmit each beam at a fundamental frequency and receive echoes at both the fundamental and harmonic frequencies. Signals from different look directions at the fundamental are spatially combined and used to form the echo tissue image 72 and signals from different look directions at the harmonic are spatially combined and used to form the harmonic contrast overlay image 74. A variation of this technique is to use the fundamental frequency signals from only one look direction to form the echo tissue background and the harmonic frequency signals from multiple look directions to form the harmonic contrast overlay image. This variation does not produce spatial compounding of the tissue image 72, but does spatially compound the harmonic contrast signals in the area 76 where the mass 78 is being diagnosed. In both of these techniques the harmonic frequencies can be separated from the fundamental frequencies by bandpass filtering.

Another approach is to use pulse inversion for harmonic separation. One way to do this is to transmit at least twice with a different modulation characteristic along each beam 82, 84, and 86. The echo ensembles along each beam direction are temporarily stored in ensemble memory 22, then combined on a spatial basis by the nonlinear signal separator 24. The different modulation of the echoes being combined (e.g., phase modulation, amplitude modulation, polarity, or a combination thereof) causes the fundamental components to be suppressed and the harmonic components to be emphasized. The fundamental signals from at least one look direction are separated by filtering or by inverse pulse inversion (e.g., instead of additively combining the differently modulated echoes, they are subtractively combined to suppress the second harmonic while emphasizing the fundamental frequency component.) The pulse inversion separated harmonic signals from the different look directions are spatially compounded by the spatial compounding processor 30 to produce the harmonic contrast image 74. Fundamental signals from multiple look directions may also be spatially compounded by the processor 30 to produce the echo tissue image 72.

In yet another embodiment the beams of the different look directions are transmitted at different transmit frequencies. For instance, the beams transmitted in look direction A can be transmitted at a nominal frequency of 1.9 MHz and the harmonics received at a frequency of 3.8 MHz and separated by bandpass filtering or pulse inversion (if multiple, differently modulated transmission are used). The beams transmitted in look direction C can be transmitted at a nominal frequency of 3.0 MHz and the echoes received at that fundamental frequency of 3.0 MHz. The beams transmitted in look direction B are done in the same manner as look direction A: transmission is done at a nominal frequency of 1.9 MHz and the harmonics received at a frequency of 3.8 MHz and separated by bandpass filtering or pulse inversion. The echo tissue image is formed from the fundamental frequency echoes received in look direction C and the harmonic contrast image is formed by spatially compounding the harmonic signals received in look directions A and B. The tissue image, while not spatially compounded, will exhibit good resolution by virtue of the fact that it is formed from the fundamental component, which is enhanced by the higher transmit frequency used.

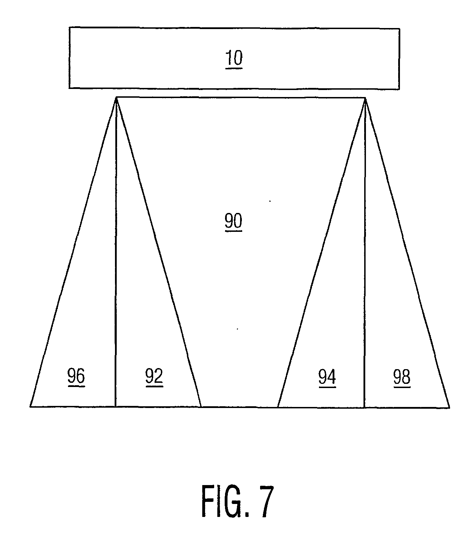

A variation of this embodiment which results in a higher frame rate of display is to transmit once along each beam 82 with one modulation characteristic in look direction A and once along each beam direction 86 with another modulation characteristic in look direction B. Pulse inversion harmonic separation is then performed by the spatial compounding process as the echoes from the differently modulation beams are combined on a spatial basis, resulting in both spatial compounding and harmonic separation. In this case the harmonic contrast image is of the trapezoidal shape as illustrated in FIGURE 7. In the central area 90 of the image field echoes are present from all three look directions and combining the echoes from two or three look directions will cause harmonic separation and a high degree of spatial compounding in this area 90. In areas 92 and 94 echoes are present from only two look directions: directions A and C for area 92 and directions B and C for area 94. Thus, with the 1.9MHz-3.0MHz-1.9MHz transmission scheme described above, a lower degree of spatial compounding will occur in these areas, but no pulse inversion harmonic separation can occur. Harmonic separation can be done in these areas by pulse inversion if each transmit beam is at 1.9MHz with different modulation characteristics so that echoes from any two or more beams can be used for pulse inversion separation. In that case, both spatial compounding and harmonic separation can be performed for areas 92 and 94. For instance, beams 82 can be transmitted at 1.9MHz with a first phase characteristic, beams 84 can be transmitted at 1.9MHz with a different amplitude characteristic, and beams 86 can be transmitted at the amplitude of beams 82 but with a different phase characteristic. Combining the echoes from some or all of these beams will result in both spatial compounding and pulse inversion nonlinear separation. In areas 96 and 98 only one echo is present from a three look direction transmit scheme, and neither spatial compounding nor pulse inversion separation can be performed in these areas.

It will be appreciated that using more than three look directions will produce additional areas and differently shaped areas for both spatial compounding and pulse inversion separation. In a constructed embodiment up to nine look directions were employed.

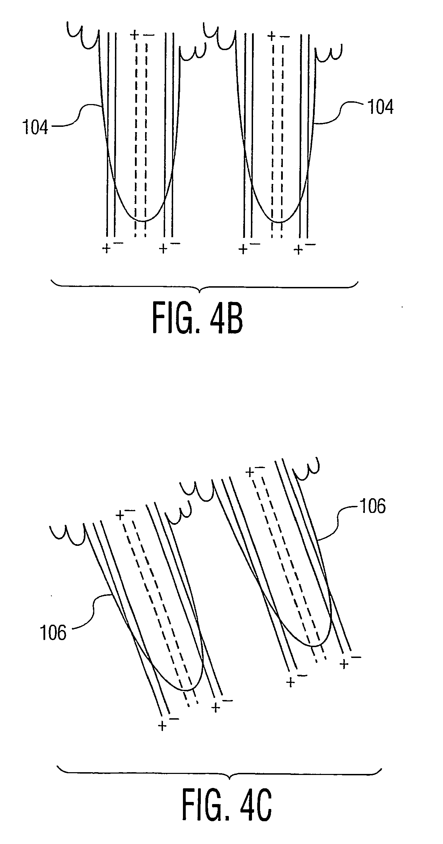

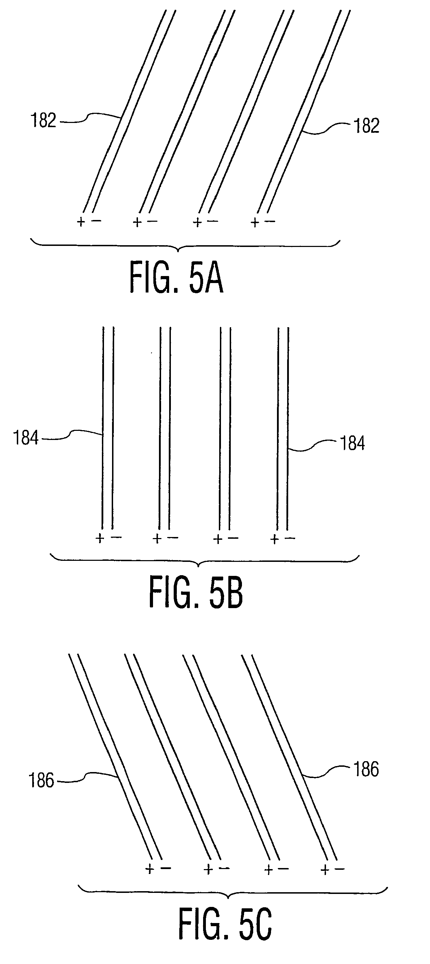

Another technique for increasing the frame rate of display is to use multiline acquisition when doing both spatial compounding and pulse inversion nonlinear signal separation. One multiline technique mentioned above is to transmit simultaneous beams in different look directions as described in the aforementioned US patent application US 2006/0293596 A1 Another multiline technique is illustrated in FIGURES 4 and 5. In FIGURES 4a-4c, beams are transmitted in three different look directions. The beams have beam profiles which encompass multiple receive beams. For instance, in FIGURE 4a each beam profile 102 has a dashed center line and encompasses two receive beams on either side of the center line. The sign "+" denotes a beam transmitted with a given modulation characteristic and the sign "-" denotes a beam transmitted with a different modulation characteristic. In the drawings the paired transmit and receive beam centers are shown parallel to each other for clarity of illustration; in practice, these beams would be aligned with each other. In FIGURE 4a, a + beam is transmitted in a given beam direction and two or more + receive beams are received in response to the transmission and held in the ensemble memory 22. A - beam is then transmitted in the beam direction and two or more - beams received in response. The aligned +/- receive beams are combined by the nonlinear signal separator 24, thereby producing two harmonic contrast beams, one on either side of the dashed center of the transmit beam 102. Thus, two or more harmonic beams are received in response to only two transmit events. This transmit/receive sequence is repeated across the aperture as shown in FIGURE 5a, producing pairs of aligned, differently modulated receive beams which can then be combined to produce pulse inversion separated harmonic contrast signals along each beam 182 in a first look direction.

This same sequence of transmission, reception and combination is then performed in a second look direction as shown by beam profiles 104 and beams 184 in FIGURES 4b and 5b. The sequence is repeated a third time in a third look direction as shown by beam profiles 106 and beams 186 in FIGURES 4c and 5c. The harmonic echo from the different look directions are then processed and combined by the spatial compounding processor 30 to form a harmonic contrast image.

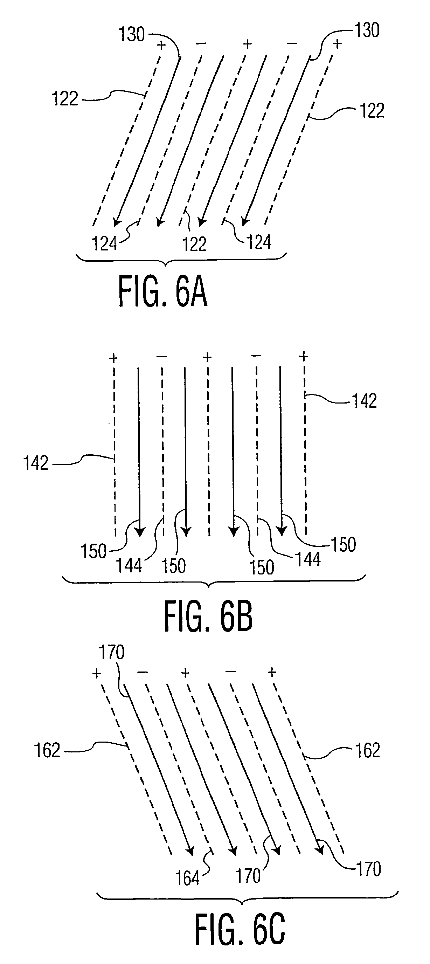

Another technique for increasing the frame rate with or without multiline reception is by the interpolation technique shown in FIGURES 6a-6c. A series of beams are transmitted and received across the aperture as shown in FIGURE 6a. These beams have alternating transmit characteristics as indicated by the + and - signs above each beam location. In this example beams 122 are modulated in a first manner and beams 124 are modulated in a second manner. The receive beams in adjacent beams 122,124 are then combined to interpolate an intermediate scanline 130. The combination of the echoes from the differently modulated beams causes harmonic separation along each scanline 130. Thus, scanlines 130 comprise a set of harmonic contrast scanlines in a first look direction.

The sequence is repeated a second time in a second look direction as shown in FIGURE 6b. Transmit beams of alternate modulation characteristics are transmitted and echoes received along each beam as shown by beams 142 and 144. Adjacent receive beams are used to interpolate intermediate scanlines 150 of separated harmonic signals acquired in a second look direction. The sequence is repeated a third time in a third look direction as shown in FIGURE 6c. Alternating transmit/receive beams 162,164 are acquired and used to interpolate intermediate separated harmonic scanlines 170. The harmonic scanlines 130, 150, 170 are then spatially compounded to produce a spatially compounded harmonic contrast image. The frame rate of display is relatively high, since each group of n scanlines is acquired by transmitting and receiving n+1 beams.

Instead of combining two receive lines (+,-) to effect pulse inversion separation, three or more laterally adjacent lines can be combined with appropriate weighting to separate the nonlinear signal components. For example, three lines (+,-,+) can be combined to form nonlinear signals at the location of the center (-) line. The adjacent line of nonlinear signals would be formed at the location of the adjacent (+) line from the combination of the (-,+,-) lines, also with appropriate weighting, and so forth.

If multiline acquisition is available the frame rate of display can be increased even higher. Each transmission of a + modulated beam centered along a + beam direction in FIGURES 6a-6c can be followed by acquisition of multiple receive beams on either side of the transmit beam center and aligned with scanline locations 130, 150, or 170. Each transmission of a - modulated beam centered along a - beam direction is followed by acquisition of multiple receive beams on either side of the transmit beam which are aligned with the scanline locations. The receive beams aligned with the same scanline location are combined to separate nonlinear signals by pulse inversion along the scanlines 130, 150, and 170. When two receive beams are received for each transmit beam, the frame rate will be the same as in the previous embodiment: n+1 beams are transmitted for each n beams in a look direction. When higher order multiline is employed, e.g., 4x, 6x or 8x multiline reception, greater frame rates will be obtained. These higher acquisition rates may be accompanied by multiline artifacts, temporal artifacts, or both, however, and may not be suitable in a given application.

## Claims

1. A method for ultrasonic imaging with contrast agents comprising:
insonifying a region of interest from a plurality of look directions by
- producing a plurality of ultrasound beams
- controlling the direction of each ultrasound beam by controlling the phasing and timing of actuation of the elements of an ultrasound array transducer
- grouping ultrasound beams to a predetermined look direction
**characterized in that** the method further comprises the steps of
- modulating transmit signals for each ultrasound beam according to its predetermined look direction
- arranging transmit signals for different look directions to be differently modulated
- separating nonlinear signal components from received echo signals; and
- combining the signals from different look directions to produce a spatially compounded image of the contrast agent in the region of interest.

2. The method of Claim 1, wherein separating nonlinear signal components further comprises separating nonlinear signal components by pulse inversion harmonic separation.

3. The method of Claim 2, wherein insonifying further comprises insonifying the region of interest with transmit signals which are differently modulated in one or more of phase, amplitude, or polarity.

4. The method of Claim 1, wherein separating the nonlinear signal components and combining the signals from different look directions are both performed by a spatial compounding processor.

5. The method of Claim 1, wherein combining further comprises combining nonlinear signal components from different look directions to produce a spatially compounded image of the contrast agent in the region of interest.

6. The method of Claim 5, wherein combining further comprises combining fundamental frequency signals from different look directions to produce a spatially compounded tissue image; and
further comprising producing an image of the spatially compounded tissue image overlaid with the spatially compounded image of the contrast agent.

7. The method of Claim 5, further comprising producing a tissue image; and
further comprising producing an image of the tissue image overlaid with the spatially compounded image of the contrast agent.

8. The method of Claim 2, wherein insonifying further comprises transmitting multiple times with different transmit characteristics along each beam direction in two or more look directions.

9. The method of Claim 8, wherein insonifying further comprises:
transmitting and receiving multiple times with different transmit characteristics along each beam direction in two look directions; and
transmitting and receiving at a fundamental frequency along each beam direction in a third look direction.

10. The method of Claim 8, wherein insonifying further comprises transmitting multiple times with different transmit characteristics along each beam direction in two or more look directions and receiving along multiple, spatially different receive beam directions in response to each transmit event.

11. The method of Claim 2, wherein insonifying further comprises transmitting spatially interleaved beams of different transmit characteristics in two or more beam directions;
wherein separating nonlinear signal components further comprises interpolating scanlines intermediate the centers of the interleaved transmit beams.

12. An ultrasonic diagnostic imaging system for imaging a region of interest containing a harmonic contrast agent comprising:
an ultrasonic array transducer which acts to transmit beams in differently steered look directions;
a transmitter coupled to the array transducer which acts to control the direction of the transmit beam by controlling the phasing and timing of actuation of the elements of the array;
a receive beamformer coupled to the array transducer to receive echo signals from microbubbles in the region of interest;
a detector coupled to the beamformer;
a spatial compounding processor, responsive to echoes from microbubbles which produces spatially compounded harmonic contrast agent image signals; and
a display coupled to the spatial compounding processor for displaying a spatially compounded harmonic contrast agent image
**characterized in that**
the transmitte is arranged to modulate the transmit signals of the transmit beams according to their look directions, and
the transmit signals for different look directions are differently modulated.

13. The ultrasonic diagnostic imaging system of Claim 12, wherein the detector further comprises a nonlinear signal separator responsive to echoes from differently modulated transmit beams which acts to separate harmonic contrast agent signals by pulse inversion.

14. The ultrasonic diagnostic imaging system of Claim 12, wherein the receive beamformer further comprises a multiline receive beamformer which acts to receive multiple, spatially different receive beams in response to one transmit event.

15. The ultrasonic diagnostic imaging system of Claim 12, wherein the detector further comprises a Doppler detector.

16. The ultrasonic diagnostic imaging system of Claim 13, wherein the detector further comprises a B mode detector which acts to detect tissue image signals,
wherein the display further acts to display a tissue image overlaid with a harmonic contrast agent image.

17. The ultrasonic diagnostic imaging system of Claim 16, wherein the detector further comprises a Doppler detector,
wherein the display further acts to display a tissue image overlaid with a color Doppler harmonic contrast agent image.

18. The ultrasonic diagnostic imaging system of Claim 12, wherein the transmitter further comprises a transmitter coupled to the array transducer which acts to differently modulate the transmit beams in one or more of phase, amplitude, or polarity.

## Patentansprüche

1. Verfahren zur Ultraschall-Bildgebung mit Kontrastmitteln, das Folgendes umfasst:
Beschallen einer interessierenden Region aus einer Vielzahl von Blickrichtungen durch
- Erzeugen einer Vielzahl von Ultraschallbündeln,
- Steuern der Richtung jedes Ultraschallbündels durch Steuern der Synchronisierung und des Zeitpunktes der Betätigung der Elemente eines Ultraschallkopfes,
- Gruppieren der Ultraschallbündel in einer vorbestimmten Blickrichtung,
**dadurch gekennzeichnet, dass** das Verfahren ferner die folgenden Schritte umfasst:
- Modulieren der Sendesignale für jedes Ultraschallbündel entsprechend seiner vorbestimmten Blickrichtung,
- Veranlassen, dass die Sendesignale für unterschiedliche Blickrichtungen unterschiedlich moduliert werden,
- Trennen der nichtlinearen Signalkomponenten von empfangenen Echosignalen und
- Kombinieren der Signale aus unterschiedlichen Blickrichtungen zur Erzeugung eines räumlich zusammengesetzten Bildes des Kontrastmittels in der interessierenden Region.

2. Verfahren nach Anspruch 1, wobei das Trennen der nichtlinearen Signalkomponenten ferner das Trennen der nichtlinearen Signalkomponenten durch die Trennung des harmonischen Anteils mit Pulsinversion umfasst.

3. Verfahren nach Anspruch 2, wobei das Beschallen ferner das Beschallen der interessierenden Region mit Sendesignalen umfasst, die hinsichtlich eines oder mehrerer der folgenden Aspekte unterschiedlich moduliert werden: Phase, Amplitude oder Polarität.

4. Verfahren nach Anspruch 1, wobei das Trennen der nichtlinearen Signalkomponenten und das Kombinieren der Signale aus unterschiedlichen Blickrichtungen beide durch einen Prozessor für räumliche Zusammensetzung (Spatial Compounding Processor) durchgeführt werden.

5. Verfahren nach Anspruch 1, wobei das Kombinieren ferner das Kombinieren der nichtlinearen Signalkomponenten aus unterschiedlichen Blickrichtungen umfasst, um ein räumlich zusammengesetztes Bild des Kontrastmittels in der interessierenden Region zu erzeugen.

6. Verfahren nach Anspruch 5, wobei das Kombinieren ferner Folgendes umfasst das Kombinieren der Grundfrequenzsignale aus unterschiedlichen Blickrichtungen umfasst, um ein räumlich zusammengesetztes Bild des Gewebes zu erzeugen, und
ferner das Erzeugen eines Abbildes des räumlich zusammengesetzten Bildes des Gewebes überlagert mit dem räumlich zusammengesetzten Bild des Kontrastmittels.

7. Verfahren nach Anspruch 5, das ferner Folgendes umfasst Erzeugen eines Gewebebildes und
ferner Erzeugen eines Abbildes des Gewebebildes überlagert mit dem räumlich zusammengesetzten Bild des Kontrastmittels.

8. Verfahren nach Anspruch 2, wobei das Beschallen ferner das mehrmalige Senden mit unterschiedlichen Sendeeigenschaften entlang jeder Schallrichtung in mindestens zwei Blickrichtungen umfasst.

9. Verfahren nach Anspruch 8, wobei das Beschallen ferner Folgendes umfasst:
mehrmaliges Senden und Empfangen mit unterschiedlichen Sendeeigenschaften entlang jeder Schallrichtung in zwei Blickrichtungen und
Senden und Empfangen mit einer Grundfrequenz entlang jeder Schallrichtung in einer dritten Blickrichtung.

10. Verfahren nach Anspruch 8, wobei das Beschallen ferner das mehrmalige Senden mit unterschiedlichen Sendeeigenschaften entlang jeder Schallrichtung in mindestens zwei Blickrichtungen und als Reaktion auf jedes Sendeereignis das Empfangen entlang mehrerer räumlich verschiedener Empfangsschallrichtungen umfasst.

11. Verfahren nach Anspruch 2, wobei das Beschallen ferner das Senden räumlich überlappter Schallbündel mit unterschiedlichen Sendeeigenschaften in mindestens zwei Schallrichtungen umfasst,
wobei das Trennen der nichtlinearen Signalkomponenten ferner das Interpolieren der zwischen den Mittelpunkten der überlappten Sendeschallbündel liegenden Abtastlinien umfasst.

12. Ultraschalldiagnose-Bildgebungssystem zum Abbilden einer interessierenden Region, das ein Kontrastmittel mit harmonischen Anteilen enthält, wobei das System Folgendes umfasst:
einen Ultraschall-Matrixschallkopf, der so funktioniert, dass er Schallbündel in unterschiedlich gelenkte Blickrichtungen sendet,
einen Sender, der mit dem Matrixschallkopf gekoppelt ist und so funktioniert, dass er die Richtung der Sendeschallbündel steuert, indem er die Synchronisierung und den Zeitpunkt der Betätigung der Elemente der Matrix steuert,
einen Empfangs-Schallbündelformer, der mit dem Matrixschallkopf gekoppelt ist und Echosignale von Mikrobläschen in der interessierenden Region empfängt,
einen Detektor, der mit dem Schallbündelformer gekoppelt ist,
einen Prozessor für räumliche Zusammensetzung (Spatial Compounding Processor), der auf Echos von Mikrobläschen reagiert und räumlich zusammengesetzte Bildsignale der harmonischen Anteile des Kontrastmittels erzeugt, und
eine Anzeige, die mit dem Prozessor für räumliche Zusammensetzung gekoppelt ist und ein räumlich zusammengesetztes Bild der harmonischen Anteile des Kontrastmittels anzeigt,
**dadurch gekennzeichnet, dass**
der Sender so ausgelegt ist, dass er die Sendesignale der Sendeschallbündel entsprechend ihren Blickrichtungen moduliert und
die Sendesignale für unterschiedliche Blickrichtungen unterschiedlich moduliert werden.

13. Ultraschalldiagnose-Bildgebungssystem nach Anspruch 12, wobei der Detektor ferner einen Trenner für nichtlineare Signale umfasst, der auf Echos von unterschiedlich modulierten Sendeschallbündeln reagiert und so funktioniert, dass er die harmonischen Anteile der Kontrastmittelsignale durch Pulsinversion trennt.

14. Ultraschalldiagnose-Bildgebungssystem nach Anspruch 12, wobei der Empfangs-Schallbündelformer ferner einen Mehrlinien-Empfangs-Schallbündelformer umfasst, der so funktioniert, dass er als Reaktion auf ein Sendeereignis mehrere räumlich unterschiedliche Empfangsschallbündel empfängt.

15. Ultraschalldiagnose-Bildgebungssystem nach Anspruch 12, wobei der Detektor ferner einen Doppler-Detektor umfasst.

16. Ultraschalldiagnose-Bildgebungssystem nach Anspruch 13, wobei der Detektor ferner einen B-Mode-Detektor umfasst, der so funktioniert, dass er Gewebebildsignale detektiert,
wobei die Anzeige ferner so funktioniert, dass sie ein Gewebebild überlagert mit einem Bild der harmonischen Anteile des Kontrastmittels anzeigt.

17. Ultraschalldiagnose-Bildgebungssystem nach Anspruch 16, wobei der Detektor ferner einen Doppler-Detektor umfasst,
wobei die Anzeige ferner so funktioniert, dass sie ein Gewebebild überlagert mit einem Farbdopplerbild der harmonischen Anteile des Kontrastmittels anzeigt.

18. Ultraschalldiagnose-Bildgebungssystem nach Anspruch 12, wobei der Sender ferner einen Sender umfasst, der mit dem Matrixschallkopf gekoppelt ist und so funktioniert, dass er die Sendeschallbündel hinsichtlich eines oder mehrerer der folgenden Aspekte unterschiedlich moduliert: Phase, Amplitude oder Polarität.

## Revendications

1. Procédé pour imagerie ultrasonique avec des agents de contraste, comprenant :
l'insonification d'une région d'intérêt à partir d'une pluralité de directions de visée en
- produisant une pluralité de faisceaux ultrasonores,
- commandant la direction de chaque faisceau ultrasonore en commandant le phasage et la synchronisation d'actionnement des éléments d'un transducteur en réseau ultrasonore,
- groupant des faisceaux ultrasonores vers une direction de visée prédéterminée,
**caractérisé en ce que** le procédé comprend en outre les étapes de :
- la modulation de signaux de transmission pour chaque faisceau ultrasonore selon sa direction de visée prédéterminée,
- l'agencement de signaux de transmission pour différentes directions de visée destinées à être modulées différemment,
- la séparation de composants de signal non linéaires à partir de signaux d'écho reçus ; et
- la combinaison des signaux à partir de différentes directions de visée pour produire une image spatialement composée de l'agent de contraste dans la région d'intérêt.

2. Procédé selon la revendication 1, dans lequel la séparation de composants de signal non linéaires comprend en outre la séparation de composants de signal non linéaires par séparation harmonique à inversion d'impulsion.

3. Procédé selon la revendication 2, dans lequel l'insonification comprend en outre l'insonification de la région d'intérêt avec des signaux de transmission qui sont modulés différemment dans une ou plusieurs parmi la phase, l'amplitude, ou la polarité.

4. Procédé selon la revendication 1, dans lequel la séparation des composants de signal non linéaires et la combinaison des signaux à partir de différentes directions de visée sont toutes les deux réalisées par un processeur de composition spatiale.

5. Procédé selon la revendication 1, dans lequel la combinaison comprend en outre la combinaison de composants de signal non linéaires à partir de différentes directions de visée pour produire une image spatialement composée de l'agent de contraste dans la région d'intérêt.

6. Procédé selon la revendication 5, dans lequel la combinaison comprend en outre la combinaison de signaux de fréquence fondamentale à partir de différentes directions de visée pour produire une image de tissu spatialement composée ; et
comprenant en outre la production d'une image de l'image de tissu spatialement composée superposée avec l'image spatialement composée de l'agent de contraste.

7. Procédé selon la revendication 5, comprenant en outre la production d'une image de tissu ; et
comprenant en outre la production d'une image de l'image de tissu superposée avec l'image spatialement composée de l'agent de contraste.

8. Procédé selon la revendication 2, dans lequel l'insonification comprend en outre la transmission de multiples fois avec différentes caractéristiques de transmission le long de chaque direction de faisceau dans deux, ou plus, directions de visée.

9. Procédé selon la revendication 8, dans lequel l'insonification comprend en outre :
la transmission et la réception de multiples fois avec différentes caractéristiques de transmission le long de chaque direction de faisceau dans deux directions de visée ; et
la transmission et la réception à une fréquence fondamentale le long de chaque direction de faisceau dans une troisième direction de visée.

10. Procédé selon la revendication 8, dans lequel l'insonification comprend en outre la transmission de multiples fois avec différentes caractéristiques de transmission le long de chaque direction de faisceau dans deux, ou plus, directions de visée et la réception le long de multiples directions de faisceau de réception spatialement différentes en réponse à chaque événement de transmission.

11. Procédé selon la revendication 2, dans lequel l'insonification comprend en outre la transmission de faisceaux spatialement entrelacés de différentes caractéristiques de transmission dans deux, ou plus, directions de faisceau ;
dans lequel la séparation de composants de signal non linéaires comprend en outre l'interpolation de lignes de balayage entre les centres des faisceaux de transmission entrelacés.

12. Système d'imagerie diagnostique ultrasonique pour imager une région d'intérêt contenant un agent de contraste harmonique, comprenant :
un transducteur en réseau ultrasonique qui sert à transmettre des faisceaux dans des directions de visée orientées différemment ;
un transmetteur couplé au transducteur en réseau qui sert à commander la direction des faisceaux de transmission en commandant le phasage et la synchronisation d'actionnement des éléments du réseau ;
un formeur de faisceau de réception couplé au transducteur en réseau pour recevoir des signaux d'écho à partir de microbulles dans la région d'intérêt ;
un détecteur couplé au formeur de faisceau ;
un processeur de composition spatiale, répondant à des échos à partir de microbulles, qui produit des signaux d'image d'agent de contraste harmonique spatialement composée ; et
un affichage couplé au processeur de composition spatiale pour afficher une image d'agent de contraste harmonique spatialement composée,
**caractérisé en ce que**
le transmetteur est agencé pour moduler les signaux de transmission des faisceaux de transmission selon leurs directions de visée, et
les signaux de transmission pour différentes directions de visée sont modulés différemment.

13. Système d'imagerie diagnostique ultrasonique selon la revendication 12, dans lequel le détecteur comprend en outre un séparateur de signaux non-linéaires répondant à des échos à partir de faisceaux de transmission modulés différemment, qui sert à séparer des signaux d'agent de contraste harmonique par inversion d'impulsion.

14. Système d'imagerie diagnostique ultrasonique selon la revendication 12, dans lequel le formeur de faisceau de réception comprend en outre un formeur de faisceau de réception multi-ligne qui sert à recevoir de multiples faisceaux de réception spatialement différents en réponse à un événement de transmission.

15. Système d'imagerie diagnostique ultrasonique selon la revendication 12, dans lequel le détecteur comprend en outre un détecteur Doppler.

16. Système d'imagerie diagnostique ultrasonique selon la revendication 13, dans lequel le détecteur comprend en outre un détecteur mode B qui sert à détecter des signaux d'image de tissu,
dans lequel l'affichage sert en outre à afficher une image de tissu superposée avec une image d'agent de contraste harmonique.

17. Système d'imagerie diagnostique ultrasonique selon la revendication 16, dans lequel le détecteur comprend en outre un détecteur Doppler,
dans lequel l'affichage sert en outre à afficher une image de tissu superposée avec une image d'agent de contraste harmonique Doppler colorée.

18. Système d'imagerie diagnostique ultrasonique selon la revendication 12, dans lequel le transmetteur comprend en outre un transmetteur couplé au transducteur en réseau qui sert à moduler différemment les faisceaux de transmission dans une ou plusieurs parmi la phase, l'amplitude, ou la polarité.
